Europäisches Patentamt

European Patent Office

Office européen des brevets

(11) Numéro de publication: **0 246 121**
**A1**

## (12) DEMANDE DE BREVET EUROPEEN

(21) Numéro de dépôt: **87400828.7**

(22) Date de dépôt: **13.04.87**

(51) Int. Cl.⁴: **A 61 M 16/00**

(30) Priorité: **15.04.86 FR 8605382**

(43) Date de publication de la demande:
**19.11.87 Bulletin 87/47**

(84) Etats contractants désignés:
**AT BE CH DE ES FR GB GR IT LI LU NL SE**

(71) Demandeur: **M.M.S. S.A.**
**89-93, Avenue Paul Vaillant Couturier**
**F-94250 Gentilly (FR)**

(72) Inventeur: **Legrand, Michel**
**3, rue Marcel Pagnol**
**F-64230 Lescar (FR)**

**Caillot, Luc**
**7, rue René Cassin**
**F-64000 Pau (FR)**

**Donnola, Jean-Michel**
**Lotissement Communal**
**F-32730 Villecomtal (FR)**

**Siretchi, Roman**
**94, rue Haxo**
**F-75020 Paris (FR)**

(74) Mandataire: **Levesque, Denys et al**
**Cabinet Beau de Loménie 55, rue d'Amsterdam**
**F-75008 Paris (FR)**

(54) **Procédé et dispositif de signalisation d'incidents dans le fonctionnement d'un respirateur.**

(57) L'invention concerne la surveillance du bon fonctionnement d'un respirateur (100) à source d'énergie unique constituée par une bouteille (2) d'oxygène sous pression, auquel est annexé un dispositif (8-11) de raccordement à un patient (3). Cette surveillance est réalisée à l'aide d'un dispositif à fonctionnement pneumatique, qui comporte un comparateur (20) à seuil ($P_s$) réglable, relié au masque (9) de raccordement au patient et suivi d'un amplificateur (21) dont le signal de sortie prend le niveau logique binaire 1 durant le temps où la pression d'insufflation dans le masque (9) est supérieure audit seuil ($P_s$), et prend l'autre niveau logique binaire 0 dans le cas contraire, et un circuit de traitement (23-26) de ce signal, qui déclenche un dispositif d'alarme (28) si la durée des intervalles de temps où ce dernier est au niveau 1 est trop grande ou trop petite par rapport à une valeur normale prédéterminée.

Fig.1

Bundesdruckerei Berlin

**Description**

## Procédé et dispositif de signalisation d'incidents dans le fonctionnement d'un respirateur

L'invention se rapporte tout d'abord à un procédé de signalisation d'incidents dans le fonctionnement d'un respirateur à source d'énergie unique constituée par la source de gaz respiratoire sous pression du respirateur, lequel permet d'appliquer une pression d'insufflation pulsatoire à un patient auquel il est raccordé.

Actuellement, les respirateurs à double source d'énergie (électrique et pneumatique), principalement utilisés dans les hôpitaux, possèdent un système d'alarme pour signaler les incidents liés soit directement au fonctionnement du respirateur, soit à des perturbations physiologiques du patient qui empêchent celui-ci de recevoir la ventilation fournie par le respirateur. Ce système d'alarme, le plus souvent actionné par un spiromètre placé sur le circuit de ventilation du patient, est électronique, le respirateur étant actionné électriquement et/ou pneumatiquement.

Par contre, les respirateurs d'urgence à source d'énergie unique, utilisés à bord des ambulances, ne possèdent pas jusqu'ici de système d'alarme. La raison réside en ceci que, pour ce type de respirateur, il est très important que le système d'alarme soit entièrement pneumatique.

La présente invention a pour but de résoudre ce problème, grâce à un procédé qui consiste essentiellement à comparer à une valeur de seuil réglable la pression d'insufflation pulsatoire fournie par le respirateur au patient et à déclencher une alarme si l'écart entre la valeur maximale de cette pression et ledit seuil dépasse une quantité prédéterminée, soit par excès, soit par défaut. Tous les incidents de fonctionnement qui se traduisent par un cycle d'insufflation présentant un maximum de pression trop élevé ou trop faible peuvent ainsi être détectés et signalés. Le procédé permet donc de surveiller le bon fonctionnement du respirateur et de son circuit de raccordement au patient, ainsi que la bonne administration du gaz respiratoire à ce dernier.

Plus particulièrement, on peut créer un signal pneumatique formé d'impulsions qui coincident avec les périodes où la pression d'insufflation est supérieure audit seuil et détecter l'apparition d'un écart excessif entre la valeur maximale de cette pression et le seuil précité en observant l'écart entre la valeur d'un paramètre représentatif desdites impulsions, tel que leur durée ou leur amplitude, et une valeur normale prédéterminée, mais réglable.

Dans une forme de réalisation, on crée, à partir dudit signal formé d'impulsions, deux signaux pneumatiques distincts en faisant subir à celui-ci une intégration de deux manières différentes, suivant deux constantes de temps respectives choisies de façon qu'une opération de coincidence logique entre ces deux signaux fournisse un résultat qui indique si la durée desdites impulsions est normale, trop grande ou trop petite. Les constantes de temps d'intégration sont avantageusement telles que l'un des deux signaux résultants reste d'un côté déterminé d'une valeur de seuil logique seulement lorsque la durée des impulsions est normale ou trop grande et reste de l'autre côté si cette durée est trop petite, et que l'autre signal est respectivement soit dudit côté déterminé soit dudit autre côté de ce seuil logique lorsque la durée des impulsions est soit normale, soit trop petite, tandis qu'il franchit alternativement ce seuil lorsque la durée des impulsions est trop grande.

L'invention a également pour objet un dispositif permettant de mettre en oeuvre le procédé défini ci-dessus. Ce dispositif, associé à un respirateur à source d'énergie unique constituée par la source de gaz respiratoire sous pression du respirateur, auquel est annexé un dispositif de raccordement à un patient, est entièrement composé d'éléments à fonctionnement pneumatique et comporte un comparateur à seuil réglable, relié au dispositif de raccordement au patient et suivi d'un amplificateur dont le signal de sortie prend un premier niveau logique binaire durant le temps où la pression d'insufflation au dispositif de raccordement est supérieure audit seuil, et prend l'autre niveau logique binaire dans le cas contraire, et un circuit de traitement de ce signal de sortie, qui détecte la durée des intervalles de temps où ce dernier est au premier niveau et déclenche une alarme si cette durée est trop grande ou trop petite par rapport à une valeur normale prédéterminée.

De préférence, ce circuit de traitement comprend deux intégrateurs dont les entrées reçoivent en commun ledit signal de sortie et dont les sorties sont reliées aux entrées d'une porte logique attaquant un dispositif d'alarme, les intégrateurs étant conçus et réglés pour que la sortie de la porte logique soit à un niveau binaire - continûment ou par intermittence - causant le déclenchement du dispositif d'alarme lorsque la durée desdits intervalles de temps est trop grande ou trop petite, et à l'autre niveau binaire, correspondant à l'inhibition du dispositif d'alarme, lorsque cette durée est normale.

Entre ladite porte logique et le dispositif d'alarme peut être interposée une deuxième porte logique comportant une entrée d'activation reliée à la source de pression et une entrée d'inhibition reliée à la sortie de la première porte logique, ce qui permet de déclencher le dispositif d'alarme en cas de baisse de pression excessive de ladite source.

Par ailleurs, l'amplificateur qui suit le comparateur peut recevoir une pression d'alimentation pulsatoire synchronisée avec les bouffées d'insufflation délivrées par le respirateur, afin de détecter toute perte de synchronisation entre la ventilation effective du patient et la cadence imposée par le respirateur.

Avantageusement, à la sortie de l'amplificateur est connecté un voyant pneumatique, qui, par ses clignotements, témoigne qualitativement du bon fonctionnement du respirateur et, quantitativement, permet le réglage initial du seuil du comparateur de pression, puis le contrôle de ce réglage.

D'autres caractéristiques et avantages de l'invention ressortiront plus clairement de la description qui va suivre, en regard des dessins annexés, d'exemples de réalisation non limitatifs utilisant, en tant que paramètre représentatif dudit signal pneumatique formé d'impulsions, respectivement la durée et l'amplitude de celles-ci.

La figure 1 représente schématiquement un dispositif selon l'invention de surveillance d'un respirateur à source d'énergie unique.

La figure 2 montre la forme de différents signaux pneumatiques qui apparaissent dans le dispositif de la figure 1.

Les figures 3 et 4 illustrent une variante de réalisation et montrent respectivement le circuit pneumatique d'alarme et la forme des signaux qui y apparaissent.

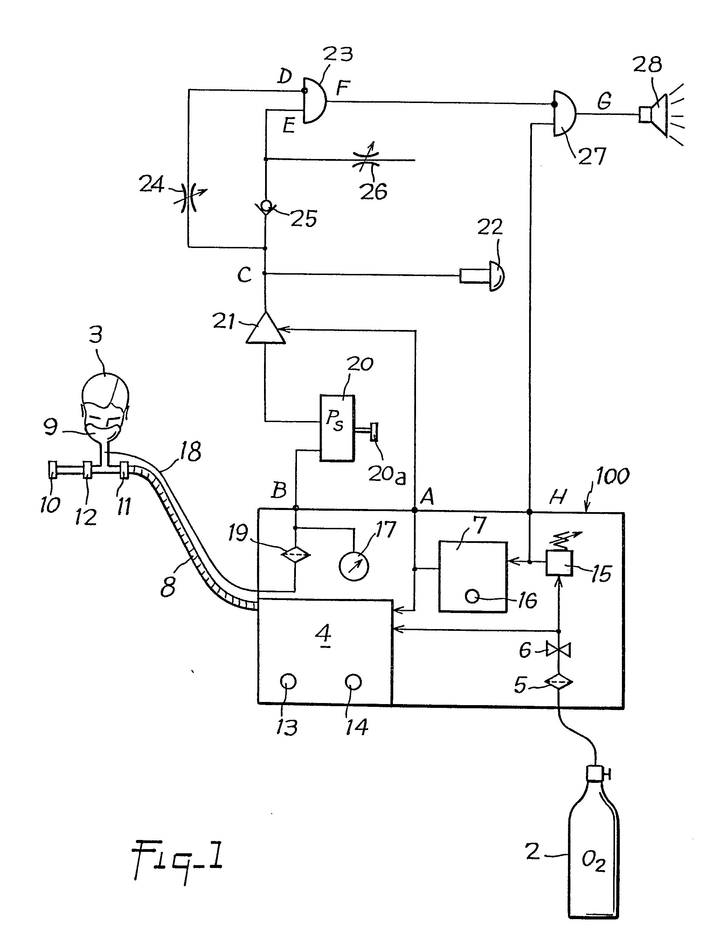

Sur la figure 1 ont été esquissés les principaux éléments d'un respirateur 100 utilisant une seule source d'énergie, constituée par une bouteille d'oxygène comprimé 2 qui fournit l'oxygène nécessaire à la ventilation respiratoire d'un patient 3.

Le respirateur 100 comprend un générateur respiratoire 4 qui, alimenté en oxygène à partir de la bouteille 2 via un filtre 5 et une vanne marche-arrêt 6 et piloté par une base de temps 7, envoie des bouffées d'insufflation d'oxygène ou de mélange air-oxygène, à la cadence de la base de temps 7, au patient 3 auquel il est relié par un tuyau 8 terminé par un masque 9 appliqué sur le visage du patient (une sonde trachéale avec ballonnet d'étanchéité gonflable peut également être utilisée pour le raccordement au patient). Le masque 9 est également relié à un dispositif d'expiration 10 permettant de fixer une pression expiratoire positive (PEP) supérieure à la pression atmosphérique. La commutation des flux gazeux d'inhalation et d'expiration est assurée par des valves 11, 12. Un bouton 13 permet de régler le volume de gaz d'inhalation débité par minute, et un autre bouton 14 permet de choisir entre l'oxygène pur et un mélange air-oxygène.

La base de temps 7 reçoit de l'oxygène comprimé via un détendeur 15. Elle comporte un bouton 16 de réglage de la fréquence de pilotage du générateur respiratoire 4.

La pression du gaz délivré au patient 3 est mesurée à l'aide d'un manomètre 17 relié au masque 9 par un petit tube auxiliaire 18, via un filtre anti-bactérien 19. En un point B relié à la sortie de ce dernier est donc disponible une pression égale à la pression régnant dans le masque 9 du patient. C'est à partir des paramètres de cette pression, variant à la cadence de la base de temps 7, qu'est effectuée la détection d'éventuels défauts de fonctionnement du respirateur 1, y compris ses éléments de liaison au patient.

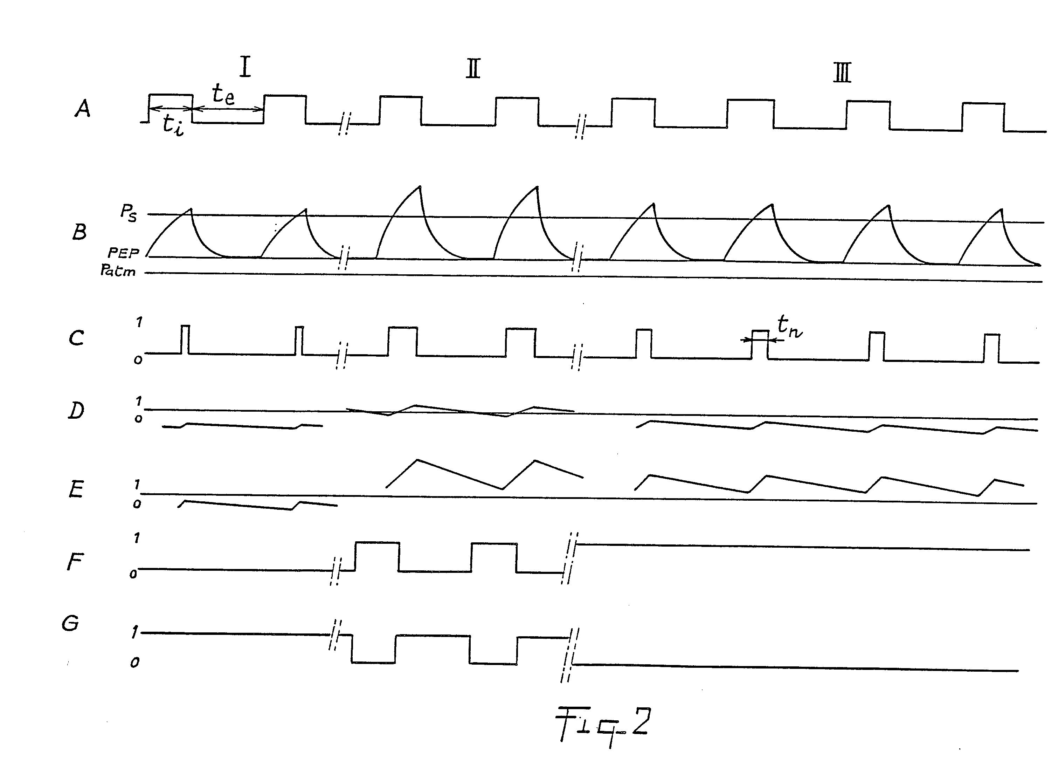

On a représenté sur la figure 2A le signal de pilotage issu de la base de temps 7 et disponible en un point A relié à la sortie de celle-ci. Ce signal détermine les cycles respiratoires successifs, composés d'une phase d'insufflation $t_i$ et d'une phase d'expiration $t_e$. A ce signal pneumatique correspond le signal disponible au point B, qui représente la pression variable relevée dans le masque 9 du patient. La forme de ce signal dépend de la présence éventuelle d'anomalies de fonctionnement et de leur nature. Le diagramme de la figure 2 illustre l'influence de deux sorts de défaut : dans le cas I existe un défaut causant une baisse anormale de pression et, dans le cas II, un défaut causant une surpression anormale, tandis que le cas III correspond à un fonctionnement normal de l'appareil en liaison avec le patient.

Ces différents cas sont distingués à l'aide d'un comparateur 20, qui reçoit le signal de pression du point B et compare son amplitude maximale à un seuil de pression $P_s$ réglable à l'aide d'un bouton 20a. Le comparateur 20 délivre des impulsions de pression durant les intervalles de temps où la pression au point B est supérieure au seuil $P_s$. Ces impulsions sont normalisées dans un amplificateur 21 qui délivre à sa sortie C des impulsions de même durée, mais de hauteur constante (voir figure 2C). On remarquera que l'amplificateur 21 est alimenté non pas par une pression de valeur constante, mais par le signal pneumatique issu de la base de temps 7 et prélevé au point A. Ainsi, ledit amplificateur n'est validé que durant les périodes d'insufflation $t_i$, de sorte que tout défaut éventuel de synchronisme entre les signaux des points A et B se manifeste dans la forme du signal à la sortie C de l'amplificateur 21 et peut être de ce fait détecté.

Un voyant pneumatique 22 est connecté au point C. Ce voyant clignote, en fonctionnement, et permet de contrôler l'existence du signal pulsatoire en ce point et d'apprécier sa forme, plus précisément son rapport cyclique, c'est-à-dire le rapport des temps où il est au niveau haut (1) et où il est au niveau bas (0). Cette information aide l'opérateur lors du réglage de la valeur de seuil $P_s$ du comparateur 20 : les durées pendant lesquelles le voyant 22 indique que le signal du point C est au niveau haut ne doivent être ni trop longues, ni trop courtes. En pratique, elles doivent être de l'ordre de $t_n = 0,2$ seconde, la valeur exacte dépendant du patient particulier secouru et de son état.

Le signal du point C est par ailleurs appliqué à un circuit de traitement par intégration, qui modifie sa forme de deux manières différentes, d'où résultent deux signaux distincts, lesquels sont appliqués aux entrées D et E d'un circuit logique pneumatique 23. Il s'agit d'une porte qui est conçue de façon que seule la présence simultanée d'un signal en D au niveau 0 et d'un signal en E au niveau 1 fasse apparaître un signal au niveau 1 à sa sortie F.

Une première voie d'intégration, reliant les points C et D, comprend une restriction 24. Il en résulte au point D (voir figure 2D) un signal assimilable à un signal de niveau 0 dans les cas I et III (impulsions courtes et moyennes au point C) et, dans le cas II (impulsions longues), un signal franchissant alternativement le niveau de séparation des zones 0 et 1.

Une seconde voie d'intégration comprend un clapet anti-retour 25 interposé entre les points C et E et n'autorisant l'écoulement que de C vers E, et une restriction 26 de fuite à l'atmosphère, reliée au point E. Le signal résultant en ce dernier point (voir figure 2E) se situe entièrement dans la zone 0 si l'on est dans le cas I et entièrement dans la zone 1 si l'on est dans le cas II ou III.

En conséquence, le signal qui apparaît à la sortie F de la porte 23 (voir figure 2F) est au niveau 0 dans le cas I, au niveau 1 dans le cas III et alternativement aux niveaux 0 et 1 dans le cas II. Ce signal est appliqué à la première entrée d'un circuit logique 27 (semblable au circuit 23) dont la seconde entrée est reliée à un point H d'où elle reçoit la pression d'alimentation appliquée à l'entrée de la base de temps 7, et qui délivre à sa sortie G un signal inverse du signal au point F (voir figure 2G), lequel excite un dispositif d'alarme sonore 28 lorsqu'il est au niveau 1.

Le dispositif décrit, conçu pour détecter les modifications de la courbe de ventilation (pression au point B), signale ainsi, par le déclenchement d'une alarme sonore - continue ou discontinue -, les différentes anomalies qui peuvent survenir dans l'utilisation du respirateur, qu'elles proviennent du circuit de raccordement au patient ou du respirateur lui-même. Une double surveillance est donc assurée par ce dispositif :

**A) Surveillance du circuit de raccordement au patient :**

1/ Une alarme sonore continue (cas I, correspondant à une baisse des maximums de ladite courbe de pression) peut signaler :

. le débranchement du tuyau 8;

. l'écrasement ou l'obturation de ce tuyau en amont de la valve 11;

. une fuite au niveau du ballonnet de la sonde trachéale utilisée pour le raccordement au patient;

. une diminution anormale de la valeur de la pression minimale PEP.

2/ Une alarme sonore discontinue (cas II, correspondant à une hausse des maximums de la courbe de pression) peut signaler :

. une obturation de la sonde trachéale;

. une augmentation anormale de la valeur de la pression minimale PEP;

. un encombrement des voies respiratoires du patient.

**B) Surveillance du bon fonctionnement du respirateur :**

1/ une alarme sonore continue (cas I) peut signaler :

. une baisse de la pression d'alimentation en gaz respiratoire;

. un défaut de la base de temps 7;

. une obturation du filtre 19;

. une fuite ou un débranchement du tube 18 de relevé de pression:

. un défaut du système d'alarme (absence de pression au point F).

2/ Une alarme sonore discontinue (cas II) peut signaler :

. un défaut de la base de temps 7;

. un défaut du dispositif de purge dont est muni le tuyau 8, empêchant la phase expiratoire.

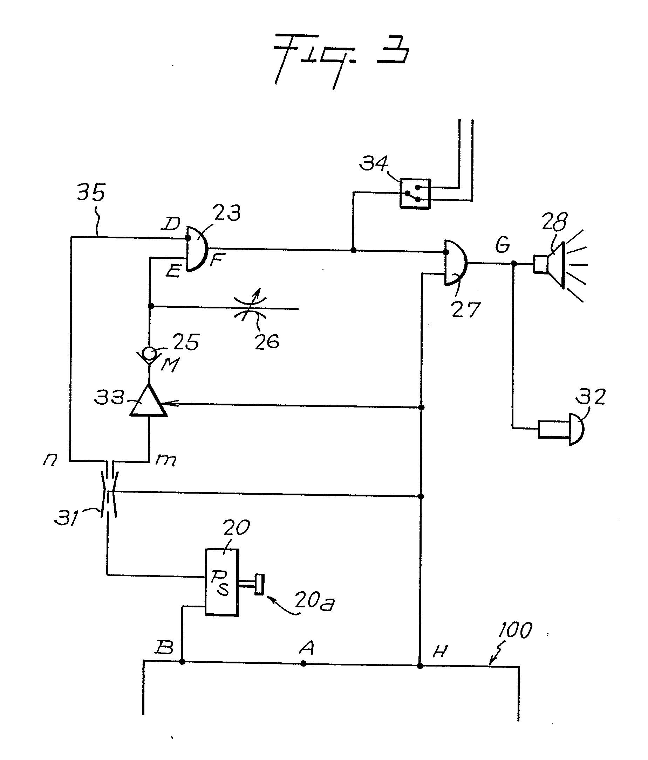

On voit sur la figure 3 une variante du circuit pneumatique d'alarme associé au respirateur 100. Dans cette variante, où le signal issu du point A n'est plus utilisé, le comparateur 20 est suivi d'un amplificateur à venturi 31 alimenté par le fluide sous pression disponible au point H. Le signal de sortie de l'amplificateur 31 apparaît dédoublé sur les deux sorties m et n qu'il comporte, qui sont reliées aux entrées D et E de la porte 23 via deux voies de traitement distinctes : le signal du point n est appliqué directement, via un conduit 35, au point D, tandis que le signal du point m est appliqué au point E par l'intermédiaire d'une voie d'intégration semblable à celle du dispositif de la figure 1 comprenant le clapet anti-retour 25 en série et la restriction 26 en dérivation vers l'atmosphère; toutefois, entre le point m et le clapet 25 est interposé un amplificateur 33 à grand gain, analogue à l'amplificateur 21 du dispositif de la figure 1, qui est alimenté par le fluide issu du point H, de pression constante.

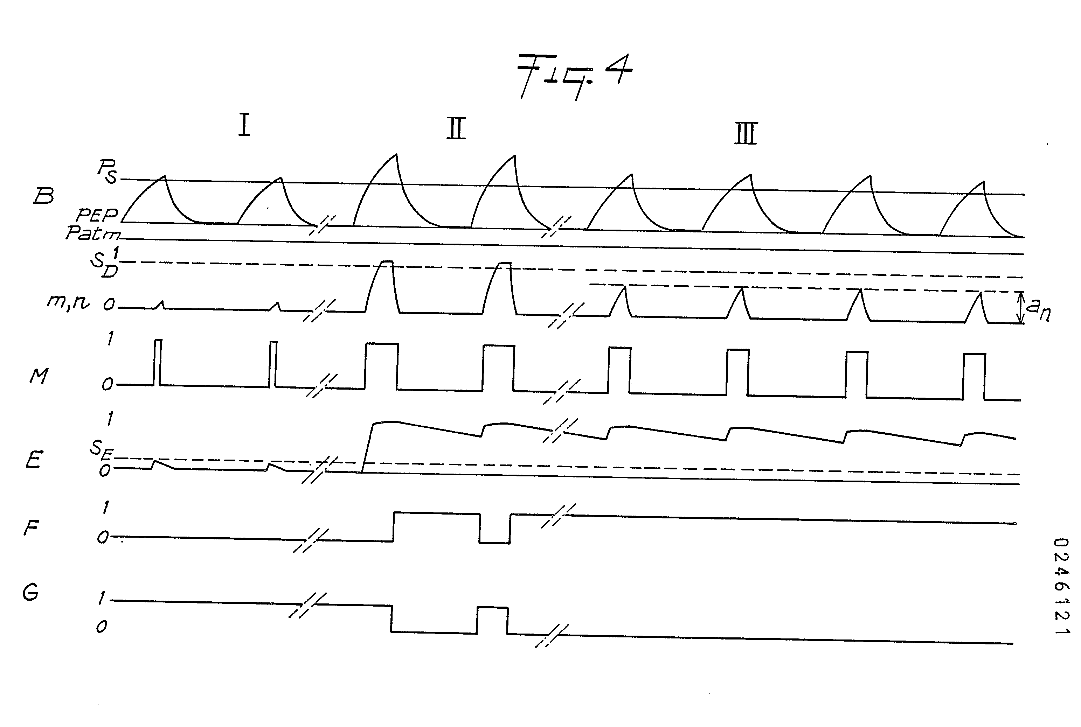

Le comparateur 20 conserve la même fonction que précédemment : il délivre des impulsions durant les intervalles de temps où la pression du point B est supérieure au seuil $P_s$. Celles-ci sont appliquées à l'entrée de refoulement du venturi 31, qui délivre sur ses deux sorties m, n un signal amplifié (cf figure 4). Le signal de la sortie m est fortement amplifié par l'amplificateur 33, à la sortie M duquel apparaît un signal rectangulaire formé de paliers aux niveaux logiques 0 ou 1. Ce dernier signal subit ensuite une intégration avec montée rapide et descente lente et c'est le signal intégré qui est appliqué à l'entrée E de la porte 23.

Dans le cas I où la pression au point B est trop faible, la pression à la sortie n du venturi 31 est très faible. Il en va de même du signal apparaissant au point E, en raison de l'étroitesse de ces impulsions, ce signal restant au-dessous du seuil de déclenchement $S_E$ de l'entrée E de la porte 23. Dès lors, celle-ci délivre en F un signal au niveau 0 et un signal d'alarme au niveau 1 apparaît au point G.

Si la pression au point B est par contre trop grande (cas II), le signal au point n dépasse le seuil de déclenchement $S_D$ de l'entrée D de la porte 23, tandis que le signal intégré au point E devient et reste nettement supérieur au niveau du seuil $S_E$. Il en résulte, à la sortie F de la porte 23, un signal d'alarme intermittent.

En fonctionnement normal, l'amplitude du signal fourni par le venturi 31 est voisin de la valeur normale $a_n$, inférieure au niveau du seuil $S_D$. Ce signal est donc trop faible pour commander l'entrée D de la porte 23, tandis que le signal intégré appliqué à l'entrée E reste supérieur au seuil $S_E$ de celle-ci (grâce au fort gain de l'amplificateur 33). Dans ces conditions, c'est un signal au niveau 1 qui apparaît à la sortie F de la porte 23, donc un signal de niveau 0 au point G, et aucun signal d'alarme n'est émis.

Une autre différence du circuit de la figure 3 par rapport à celui de la figure 1 réside dans le mode de connexion du voyant utilisé. Ce voyant, qui porte ici la référence 32, est connecté au point G, en parallèle avec le dispositif d'alarme sonore 28 et fournit maintenant une alarme visuelle, utile en particulier dans les ambiances à fort niveau de bruit. On remarquera que si, dans le circuit de la figure 1, le comparateur 20 doit être réglé pour obtenir des clignotements de courte durée du voyant 22, il doit maintenant être réglé pour ne pas obtenir de clignotements du voyant 32.

Le circuit de la figure 3 est complété par un manocontact 34, dont l'entrée est connectée au point F. Il comporte un contact électrique qui change de position lorsque le signal au point F change de niveau logique. Ce contact électrique, normalement ouvert en présence de ce signal au niveau 1, peut être utilisé pour commander un rappel d'alarme, éventuellement à distance du respirateur. On notera que le manocontact 34 est au surplus susceptible de détecter et de signaler un défaut survenant sur l'alimentation pneumatique du respirateur (dû par exemple à une rupture du tuyau de liaison à la bouteille d'oxygène sous pression), alors que les dispositifs d'alarme 28, 32 deviennent, de ce fait, inopérants.

## Revendications

1. Procédé de signalisation d'incidents dans le fonctionnement d'un respirateur à source d'énergie unique constituée par la source de gaz respiratoire sous pression du respirateur, lequel permet d'appliquer une pression d'insufflation pulsatoire à un patient auquel il est raccordé, dans lequel on compare à une valeur de seuil ($P_S$) réglable ladite pression d'insufflation pulsatoire et on déclenche une alarme si l'écart entre la valeur maximale de cette pression et ledit seuil dépasse une quantité prédéterminée, soit par excès, soit par défaut, caractérisé par le fait que l'on crée un signal pneumatique formé d'impulsions qui coincident avec les périodes où la pression d'insufflation est supérieure audit seuil ($P_S$) et que l'on détecte l'apparition d'un écart excessif entre la valeur maximale de cette pression et le seuil en observant l'écart entre un paramètre représentatif desdites impulsions et une valeur normale ($t_n$, $a_n$) prédéterminée de ce paramètre, cette valeur étant réglable.

2. Procédé selon la revendication 1, caractérisé par le fait que, à partir dudit signal formé d'impulsions, on crée deux signaux pneumatiques distincts en faisant subir à celui-ci un traitement de deux manières différentes choisies de façon qu'une opération de coincidence logique entre ces deux signaux fournisse un résultat qui indique si la valeur du paramètre représentatif desdites impulsions est normale, trop grande ou trop petite.

3. Procédé selon la revendication 2, caractérisé par le fait que l'on applique audit signal un traitement de deux manières telles que l'un des deux signaux résultants reste d'un côté déterminé (1) d'une valeur de seuil seulement lorsque la valeur du paramètre des impulsions est normale ou trop grande et reste de l'autre côté (0) si cette valeur est trop petite, et que l'autre signal est respectivement soit dudit côté déterminé (1) soit dudit autre côté (0) de ce seuil lorsque la valeur du paramètre des impulsions est soit normale, soit trop petite, tandis qu'il franchit alternativement ce seuil lorsque la valeur du paramètre des impulsions est trop grande.

4. Procédé selon la revendication 3, caractérisé par le fait que le paramètre est la durée desdites impulsions, que l'on compare à une durée normale ($t_n$) prédéterminée, de valeur réglable.

5. Procédé selon la revendication 4, caractérisé par le fait que les deux signaux pneumatiques distincts sont créés en faisant subir audit signal formé d'impulsions une intégration de deux manières différentes, suivant deux constantes de temps respectives choisies de façon qu'une opération de coincidence logique entre ces deux signaux fournisse un résultat qui indique si la durée desdites impulsions est normale, trop grande ou trop petite.

6. Procédé selon la revendication 5, caractérisé par le fait que les constantes de temps d'intégration son telles que l'un des deux signaux résultants reste d'un côté déterminé (1) d'une valeur de seuil logique seulement lorsque la durée des impulsions est normale ou trop grande et reste de l'autre côté (0) si cette durée est trop petite, et que l'autre signal est respectivement soit dudit côté déterminé (1) soit dudit autre côté (0) de ce seuil logique lorsque la durée des impulsions est soit normale, soit trop petite, tandis qu'il franchit alternativement ce seuil lorsque la durée des impulsions est trop grande.

7. Procédé selon la revendication 3, caractérisé par le fait que le paramètre est l'amplitude desdites impulsions, que l'on compare à une amplitude normale ($a_n$) prédéterminée, de valeur réglable.

8. Procédé selon la revendication 7, caractérisé par le fait que les deux signaux pneumatiques distincts sont créés en faisant subir audit signal formé d'impulsions une forte amplification, puis une intégration suivant une constante de temps choisie de façon qu'une opération de coincidence logique entre ledit signal avant amplification et intégration et ledit signal après amplification et intégration fournisse un résultat qui indique si l'amplitude desdites impulsions est normale, trop grande ou trop petite.

9. Procédé selon la revendication 8 caractérisé par le fait que la constante de temps d'intégration est telle que l'un des deux signaux reste d'un côté déterminé (1) d'une première valeur de seuil ($S_E$) seulement lorsque l'amplitude des impulsions est normale ou trop grande et reste de l'autre côté (0) si cette amplitude est trop petite, et que l'autre signal est respectivement soit dudit côté déterminé (1) et dépasse une deuxième valeur de seuil ($S_D$), soit dudit autre côté (0) et au-dessous de ce seuil lorsque l'amplitude des impulsions est soit normale, soit trop petite, tandis qu'il franchit alternativement ce seuil lorsque l'amplitude des impulsions est trop grande.

10. Dispositif de signalisation d'incidents dans le fonctionnement d'un respirateur à source d'énergie unique constituée par la source de gaz respiratoire sous pression du respirateur, auquel est annexé un dispositif de raccordement à un patient, mettant en oeuvre le procédé selon l'une quelconque des revendications 4 à 6,

caractérisé par le fait qu'il est entièrement composé d'éléments à fonctionnement pneumatique et comporte un comparateur (20) à seuil ($P_s$) réglable, relié au dispositif (9) de raccordement au patient et suivi d'un amplificateur (21) dont le signal de sortie prend un premier niveau logique binaire (1) durant le temps où la pression d'insufflation au dispositif de raccordement (9) est supérieure audit seuil ($P_s$), et prend l'autre niveau logique binaire (0) dans le cas contraire, et un circuit de traitement (24, 25, 26) de ce signal de sortie, qui détecte la durée des intervalles de temps où ce dernier est au premier niveau (1) et déclenche une alarme si cette durée est trop grande ou trop petite par rapport à une valeur normale prédéterminée.

11. Dispositif selon la revendication 10, caractérisé par le fait que le circuit de traitement comprend deux intégrateurs (24; 25, 26) dont les entrées reçoivent en commun ledit signal de sortie et dont les sorties sont reliées aux entrées (D, E) d'une porte logique (23) attaquant un dispositif d'alarme (28), les intégrateurs étant conçus et réglés pour que la sortie (F) de la porte logique (23) soit à un niveau binaire (0) - continûment ou par intermittence - causant le déclenchement du dispositif d'alarme (28) lorsque la durée desdits intervalles de temps est trop grande ou trop petite, et à l'autre niveau binaire (1) correspondant à l'inhibition du dispositif d'alarme, lorsque cette durée est normale.

12. Dispositif selon la revendication 10 ou 11, caractérisé par le fait que l'amplificateur (21) qui suit le comparateur (20) reçoit une pression d'alimentation pulsatoire synchronisée avec les bouffées d'insufflation délivrées par le respirateur.

13. Dispositif selon l'une quelconque des revendications 10 à 12, caractérisé par le fait qu'à la sortie de l'amplificateur (21) est connecté un voyant pneumatique (22).

14. Dispositif de signalisation d'incidents dans le fonctionnement d'un respirateur à source d'énergie unique constituée par la source de gaz respiratoire sous pression du respirateur, auquel est annexé un dispositif de raccordement à un patient, mettant en oeuvre le procédé selon l'une quelconque des revendications 7 à 9, caractérisé par le fait qu'il est entièrement composé d'éléments à fonctionnement pneumatique et comporte un comparateur (20) à seuil ($P_s$) réglable, relié au dispositif (9) de raccordement au patient et suivi d'un amplificateur (31) dont le signal de sortie a une amplitude variable, et un circuit de traitement (35; 33, 25, 26) de ce signal de sortie, qui détecte la valeur de l'amplitude de ce dernier par rapport à un niveau normal ($a_n$) et déclenche une alarme si cette valeur est trop grande ou trop petite par rapport à ce niveau normal prédéterminé.

15. Dispositif selon la revendication 14, caractérisé par le fait que le circuit de traitement comprend une première voie directe (35) et une deuxième voie d'amplification à grand gain et d'intégration (33, 25, 26) dont les entrées reçoivent en commun ledit signal de sortie et dont les sorties sont reliées aux entrées (D, E) d'une porte logique (23) attaquant un dispositif d'alarme (28), les deux voies étant conçues et réglées pour que la sortie (F) de la porte logique (23) soit à un niveau binaire (0) -continûment ou par intermittence - causant le déclenchement du dispositif d'alarme (28) lorsque l'amplitude dudit signal de sortie est trop grande ou trop petite, et à l'autre niveau binaire (1), correspondant à l'inhibition du dispositif d'alarme, lorsque cette amplitude est normale.

16. Dispositif selon la revendication 11 ou 15, caractérisé par le fait qu'entre ladite porte logique (23) et le dispositif d'alarme (28) est interposée une deuxième porte logique (27) comportant une entrée d'activation reliée (en H) à la source de pression et une entrée d'inhibition reliée à la sortie (F) de la première porte logique (23).

17. Dispositif selon l'une quelconque des revendications 11 à 13, 15 et 16, caractérisé par le fait que le dispositif d'alarme (28) est conçu pour émettre une alarme sonore et qu'un voyant pneumatique (32) est connecté en parallèle avec ledit dispositif d'alarme (28).

18. Dispositif selon l'une quelconque des revendications 11 à 13 et 15 à 17, caractérisé par le fait qu'un manocontact (34) est connecté à la sortie (F) de la porte (23) et déclenche une alarme en cas de manque de pression à cette sortie.

0246121

Fig-1

Fig.2

0246121

Fig. 3

Fig. 4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | US-A-3 867 934  (L. OLIVIER)<br>* Figure 1; colonne 6, lignes 18-30; colonne 10; lignes 6-12 * | 1,10 | A 61 M  16/00 |
| Y | US-A-4 381 774  (P. SCHREIBER et al.)<br>* Figure 2; colonne 10, ligne 34 - colonne 11, ligne 13 * | 1,10 | |
| A | FR-A-2 202 677  (SOCIETE MINERVE S.A.)<br>* Figures; page 2, ligne 26 - page 5, ligne 36 * | 1 | |
| A | FR-A-2 133 850  (VEB KOMBINAT MEDIZIN- UND LABORTECHNIK LEIPZIG)<br>* Figures; page 8, ligne 39 - page 9, ligne 13 * | 1 | **DOMAINES TECHNIQUES RECHERCHES (Int. Cl.4)**<br><br>A 61 M |

Le présent rapport de recherche a été établi pour toutes les revendications

| Lieu de la recherche | Date d'achèvement de la recherche | Examinateur |
|---|---|---|
| LA HAYE | 13-07-1987 | VEREECKE A. |